# EUROPEAN PATENT SPECIFICATION

(11) **EP 2 585 184 B1**
(45) Date of publication and mention of the grant of the patent: **26.07.2017**
(21) Application number: 11727440.7
(22) Date of filing: 22.06.2011
(51) Int. Cl.: C07D 307/48, C07D 307/50, B01D 11/04

(54) **PROCESS FOR SEPARATING FURFURAL FROM A LIQUID AQUEOUS PHASE COMPRISING FURFURAL AND ONE OR MORE ORGANIC ACIDS**
VERFAHREN ZUM TRENNEN VON FURFURAL AUS EINER FLÜSSIGWÄSSRIGEN PHASE MIT FURFURAL UND EINER ODER MEHR ORGANISCHEN SÄUREN
PROCÉDÉ DE SÉPARATION DU FURFURAL À PARTIR D'UNE PHASE AQUEUSE LIQUIDE CONTENANT DU FURFURAL ET UN OU PLUSIEURS ACIDES ORGANIQUES

(30) Priority: 22.06.2010 EP 10166868
(43) Date of publication of application: 01.05.2013
(73) Proprietor: Shell Internationale Research Maatschappij B.V., 2596 HR Den Haag (NL)
(72) Inventor: HAAN, Johannes Pieter, NL-1031 HW Amsterdam (NL)
(74) Representative: Matthezing, Robert Maarten
(86) International application number: PCT/EP2011/060409
(87) International publication number: WO 2011/161141

(56) References cited:
- DE-A1-102008 009 933
- FR-A1- 2 858 618
- GB-A- 876 463
- US-A- 3 869 357
- US-A- 4 533 743
- US-B1- 6 441 202

## Description

### TECHNICAL FIELD OF THE INVENTION

The present invention relates to a process for separating furfural from a liquid aqueous phase comprising furfural and one or more organic acids.

### BACKGROUND TO THE INVENTION

Furfural, also known as Furan-2-carbaldehyde, is a valuable intermediate in the production of sustainable biofuels. Biofuels are combustible fuels, that can be derived from biological sources. The use of such biofuels results in a reduction of greenhouse gas emissions.

Furfural can conveniently be produced from biomass. It is for example produced in the liquefaction of lignocellulosic material.

After the liquefaction of lignocellulosic material it is desirable to separate furfural from the total aqueous product produced. Separation of the furfural by distillation, however, is problematic as furfural can form azeotropes with the water in the total aqueous product.

Alternative approaches to recovering furfural include liquid-liquid extraction processes.

US4533743 describes a process for the production of furfural. It describes that state of the art biomass acid hydrolysis processing techniques can breakdown pentosans, a major constituent of biomass hemicellulose, into pentoses. Hot pentose is subsequently reacted in the presence of a mineral acid catalyst in a plug flow reactor at a temperature in the range from 220°C to 300°C to produce furfural. The produced furfural is optionally extracted using an essentially water immiscible furfural solvent which does not form an azeotrope with furfural.

As suitable solvents amongst others higher boiling point aromatics, such as diethylbenzene, dipropylbenzene, dimethylethylbenzene, butylbenzene, tetralin and isophorone; aromatics, such as toluene; halogenated aromatics; and also halogenated alkanes are mentioned.

US 6,441,202 describes a method to produce sugars by acidic hydrolysis of biomass and subsequently subject the sugars to dehydration to form a hydrolysate comprising heterocyclic compounds such as furfural and hydroxymethylfurfural and acid. Subsequently the heterocyclic compounds are extracted from the hydrolysate by a hydrocarbon. The acid may include an organic or inorganic acid, such as for example sulfuric acid and the hydrocarbon may for example be toluene.

FR2411184 also describes a process for the preparation of furfural. It describes submitting of a sugar solution to acid dehydration to convert xylose into furfural. The furfural is extracted with a solvent. As suitable solvents amongst others toluene, xylene, methylnaphthalene and benzaldehyde are mentioned.

J. Croker et al. describe a process for liquid extraction of furfural from an aqueous solution (see their article "liquid extraction of furfural from aqueous solution" by John R. Croker and Ron G. Bowrey, Ind. Eng. Chem. Fundam. 1984, vol.23, pages 480-484). They describe extraction for water-furfural methyl isobutyl ketone; water-furfural-isobutyl acetate and water-furfural-toluene systems.

DE 10 2008 009933 disclsoes a process for the extraction of 5-hydroxymethylfufural from an ionic liquid.

In the above prior art methods for extracting furfural, however, the starting solution is a solution comprising only a limited amount of components, such as for example furfural, water and optionally sugars and/or an acid such as sulfuric acid.

### SUMMARY OF THE INVENTION

It has now been advantageously found that furfural can also be selectively extracted by liquid-liquid extraction from a complex starting solution, comprising not only furfural, water and optionally sugars, but also a complex combination of one or more organic acids chosen from the group of levulinic acid, acetic acid, formic acid and mixtures thereof.

The present invention therefore provides a process for separating furfural from a liquid aqueous phase comprising furfural and one or more organic acids chosen from the group of levulinic acid, acetic acid, formic acid and mixtures thereof, which process comprises a step a) extracting furfural from the liquid aqueous phase into a liquid aromatic phase to obtain a liquid organic phase comprising furfural characterized in that the liquid aromatic phase comprises aromatic hydrocarbon compounds including a mixture of a C₁₋₆ alkyl substituted naphthalene with a C₁₋₆ substituted benzene or comprising a mixture of naphthalene and 1-methylnaphthalene.

It has presently been found that contacting the liquid aqueous phase comprising furfural and one or more organic acids chosen from the group of levulinic acid, acetic acid, formic acid and mixtures thereof with a liquid aromatic phase comprising said aromatic hydrocarbon compounds allows one to selectively extract furfural from the liquid aqueous phase into the liquid aromatic phase, leaving the aqueous phase depleted in furfural, but still rich in organic acid.

The present invention, therefore, makes it possible to directly extract furfural from a liquid aqueous phase, comprising furfural and one or more organic acids chosen from the group of levulinic acid, acetic acid, formic acid and mixtures thereof, formed by acid catalyzed hydrolysis of biomass.

The selective extraction of furfural from organic acids in an aqueous environment using these aromatic hydrocarbon compounds has not previously been suggested.

### BRIEF DESCRIPTION OF THE DRAWINGS

Figure 1 shows a schematic diagram of a process for extracting furfural according to the invention.
Figure 2 shows a schematic diagram of a process for extracting furfural according to the invention with recovery of the extraction solvent.

### DETAILED DESCRIPTION OF THE INVENTION

In the process according to the present invention, furfural is extracted from a liquid aqueous phase comprising furfural and one or more organic acids chosen from the group of levulinic acid, acetic acid, formic acid and mixtures thereof.

By a liquid phase is herein understood a phase that is liquid at extraction temperature and pressure. By a liquid aqueous phase is herein understood a liquid phase comprising at least water.

In one embodiment, the liquid aqueous phase is produced by acid catalyzed hydrolysis of biomass, preferably of lignocellulosic material. Preferably the liquid aqueous phase is obtained by liquefaction of lignocellulosic material. As used herein, lignocellulosic material refers to a material comprising lignin, cellulose and hemicellulose.

Without wishing to be bound to any kind of theory it is believed that liquefaction of lignocellulosic material can comprise cleavage of covalent linkages in the cellulose, hemicellulose and lignin present and/or cleavage of covalent linkages between lignin, hemicelluloses and/or cellulose. As a result acids such as formic acid, acetic acid and/or levulinic acid can be formed together with sugars and optionally lignin degradation products. Cellulose present in the lignocellulosic material can be converted into a sugar containing six carbon atoms which is degraded in the presence of the acid catalyst to produce hydroxymethylfurfural and then further hydrolyzed to give levulinic acid. Hemicellulose present in the lignocellulosic material can be converted into a five carbon sugar which breaks down to give furfural.

The lignocellulosic material may be obtained from a variety of plants and plant materials including agricultural wastes, forestry wastes and sugar processing residues. Examples of suitable lignocellulosic materials include agricultural wastes such as corn stover, soybean stover, corn cobs, rice straw, rice hulls, oat hulls, corn fibre, cereal straws such as wheat, barley, rye and oat straw; grasses; forestry products such as wood and wood-related materials such as sawdust; sugar processing residues such as bagasse and beet pulp; or mixtures thereof.

Liquefaction of the lignocellulosic material can be carried out in any manner known to the skilled person to be suitable for such a purpose. The liquefaction of lignocellulosic material can suitably comprise hydrolyzing the lignocellulosic material in the presence of an acid catalyst. Examples of suitable acid catalysts include mineral acids such as sulphuric acid, paratoluene-sulphonic-acid, nitric acid, hydrochloric acid, phosphoric acid and/or mixtures thereof; and organic acids such as oxalic acid, formic acid, lactic acid, citric acid, trichloracetic acid and/or mixtures thereof.

The temperature applied during hydrolysis may vary widely and preferably ranges from 100°C to 300°C, more preferably from 150°C to 250°C,
In one embodiment, a lignocellulosic material is hydrolyzed in the presence of an acid catalyst acid to prepare a liquid aqueous phase comprising furfural and one or more organic acids chosen from the group of levulinic acid, acetic acid, formic acid and mixtures thereof, which liquid aqueous phase is subsequently used directly as the starting liquid aqueous phase of the process according to the invention in the absence of additional recovery or concentration steps.

The process of the invention is therefore particularly advantageous for the extraction of furfural from a liquid aqueous phase comprising furfural and one or more organic acids chosen from the group of levulinic acid, acetic acid, formic acid and mixtures thereof, which liquid aqueous phase is produced by acid catalyzed hydrolysis of a lignocellulosic material.

The liquid aqueous phase comprises preferably more than or equal to 0.1 wt%, more preferably more than or equal to 0.5 wt%, and most preferably more than or equal to 1.0wt% furfural and preferably less than or equal to 30.0 wt%, more preferably less than or equal to 20wt% and most preferably less than or equal to 10.0 wt% furfural, based on the total weight of the liquid aqueous phase.

The liquid aqueous phase further comprises preferably more than or equal to 0.1 wt%, more preferably more than or equal to 0.5 wt%, and most preferably more than or equal to 1.0 wt% of one or more organic acids chosen from the group of levulinic acid, acetic acid, formic acid and mixtures thereof and preferably less than or equal to 30.0 wt%, more preferably less than or equal to 25.0 wt% and most preferably less than or equal to 20.0 wt% of one or more organic acids chosen from the group of levulinic acid, acetic acid, formic acid and mixtures thereof, based on the total weight of the liquid Preferably the liquid aqueous phase comprises essentially only organic acids chosen from the group of levulinic acid, acetic acid, formic acid and mixtures thereof. In a most preferred embodiment the liquid aqueous phase comprises at least levulinic acid.

In one embodiment, the liquid aromatic phase comprises aromatic hydrocarbon compounds including a mixture of a C₁₋₆ alkyl substituted naphthalene with a C₁₋₆ alkyl substituted benzene. The term "alkyl" includes both straight chain and branched chain alkyl groups. The benzene or naphthalene ring is preferably substituted with one to four, more preferably one or two alkyl groups. Preferably the alkyl group contains 2 to 4 carbon atoms.

Preferably the liquid aromatic phase comprises aromatic hydrocarbon compounds having a higher boiling point than furfural. The use of aromatic hydrocarbon compounds having a higher boiling point than furfural advantageously facilitates recycling of the aromatic hydrocarbon compound, reducing the amount of energy required and thereby improving the cost effectiveness of the process of the invention.

It has been found that although toluene (methyl benzene) can selectively extract furfural from a liquid aqueous phase comprising furfural and one or more organic acids, the fact that it has a boiling point (111°C) which is below that of furfural (162°C) makes it costly to recycle.

Naphthalene has a boiling point (218°C) which is higher than that of furfural, indicating that it should be well suited to extracting furfural from an aqueous phase comprising furfural. However, the practical applications of naphthalene for use as an extraction solvent (i.e. as liquid aromatic phase) according to the present invention are limited as naphthalene is a solid below 80°C, meaning that liquid/liquid extractions using naphthalene are not feasible below this temperature.

In one embodiment, the liquid aromatic phase comprises a mixture of naphthalene and 1-methylnaphthalene. By dissolving naphthalene in 1-methylnaphthalene, which is a liquid at room temperature with a boiling point of 241°C, high extraction selectivity can be achieved whilst avoiding the practical problems associated with using naphthalene alone. In a preferred embodiment the liquid aromatic phase consists essentially of a mixture of naphthalene and 1-methylnaphthalene. Preferably a mixture is used with a weight ratio of naphthalene to 1-methylnaphthalene in the range of 1:5 to 5:1, more preferably 1:4 to 4:1, and most preferably 1:2 to 2:1.

The liquid aromatic phase comprises preferably more than or equal to 40 wt%, more preferably more than or equal to 50 wt% and most preferably more than or equal to 60 wt% of one or more aromatic hydrocarbon compounds, based on the total weight of liquid aromatic phase. For practical purposes the liquid aromatic phase may comprise equal to or less than 100wt% of one or more aromatic hydrocarbon compounds, based on the total liquid aromatic phase.

The liquid aromatic phase further comprises preferably less than 40 wt% of non-aromatic hydrocarbon compounds, more preferably less than 10 wt% of non-aromatic hydrocarbon compounds, based on the total liquid aromatic phase. Most preferably the liquid aromatic phase is essentially free of non-aromatic hydrocarbon compounds.

Examples of such non-aromatic hydrocarbon compounds include (branched-)paraffins, (branched-)olefins and naphtenics.

The furfural can be extracted from the liquid aqueous phase comprising furfural and one or more organic acids by contacting the liquid aqueous phase with the liquid aromatic phase comprising one or more aromatic hydrocarbon compounds. The liquid aromatic phase can be added to the liquid aqueous phase or the liquid aqueous phase can be added to the liquid aromatic phase.

Preferably the liquid aromatic phase is added to the liquid aqueous phase. Contacting of the liquid aqueous phase and the liquid aromatic phase can be carried out batchwise or continuously. When carried out continuously the contacting of the liquid aqueous phase and the liquid aromatic phase may be carried out counter-currently, cocurrently or cross-currently with respect to each other.

Preferably, the liquid aqueous phase and the liquid aromatic phase are contacted with each other counter-currently, more preferably with the liquid stream with the highest density entering at the top of an extraction column and the liquid stream with the lower being fed in at the bottom of the extraction column. Alternatively several mixer/settler units in series can be used.

Upon contact of the liquid aqueous phase and the liquid aromatic phase, two phases can be formed. Furfural present in the liquid aqueous phase is selectively extracted into the liquid aromatic phase, forming a liquid organic phase and leaving the liquid aqueous phase depleted in furfural. Organic acid(s) present in the liquid aqueous phase remain in the liquid aqueous phase, leaving the liquid aqueous phase rich in organic acid(s).

By a liquid organic phase is understood a liquid phase comprising one or more organic compounds. The liquid organic phase formed comprises furfural and one or more aromatic hydrocarbon compounds. The formed liquid organic phase comprises preferably in the range from 0.2 wt% to 40.0 wt%, more preferably in the range from 1.0 wt% to 20.0 wt% of furfural based on the total weight of liquid organic phase.

Preferably, the liquid aqueous phase comprising furfural and one or more organic acids and the liquid aromatic phase comprising one or more aromatic hydrocarbon compounds are contacted at a ratio of the liquid aqueous phase to the liquid aromatic phase in the range of from 2:1 to 1:5.

The pressure and temperature can be selected such that the liquid aqueous phase and liquid aromatic phase remain in the liquid state. Preferably the process according to the invention is carried out at a temperature of less than or equal to 200°C, more preferably a temperature of less than or equal to 150°C, most preferably a temperature of less than or equal to 90°C and a temperature of more than or equal to 15°C, more preferably a temperature of more than or equal to 20°C.

The process according to the invention is further preferably conducted at a pressure of less than or equal to 25 bar absolute, more preferably less than or equal to 10 bar, most preferably at ambient pressure (1 bar absolute).

After extraction of the furfural from the liquid aqueous phase into the liquid aromatic phase, the formed furfural-depleted liquid aqueous phase may be separated and removed from the formed liquid organic phase by conventional techniques such as gravitational separation (for example settling).

The formed furfural-depleted liquid aqueous phase is preferably removed from the formed liquid organic phase and preferably recycled and re-used, for example in acid-catalyzed hydrolysis of lignocellulose to provide more furfural in liquid aqueous solution.

The aromatic hydrocarbon compounds are preferably recovered from the formed liquid organic phase and recycled for use in the extraction process.

In a preferred embodiment the process according to the invention subsequently comprises a further step wherein the furfural is retrieved from the liquid organic phase.

Separation of the extracted furfural from the liquid organic phase into which it is extracted can be achieved by any technique known to be suitable for this purpose. A preferred technique is distillation. An advantage of using one or more aromatic hydrocarbon compounds having a higher boiling point than furfural is that the extracted furfural can be evaporated instead of the aromatic hydrocarbon compound(s). As the furfural is generally present in a smaller amount than the aromatic hydrocarbon compound(s), this requires less energy. Furthermore, the use of one or more higher boiling point aromatic hydrocarbon compound(s) allows a lower ratio of the liquid aqueous phase to liquid aromatic phase to be used in the process of the invention without incurring a large energy penalty in the recovery step. This means that more aromatic hydrocarbon compound per unit of furfural is available in the extraction tower to remove furfural from the liquid aqueous phase. Additionally, separation of furfural from the liquid organic phase by removal of the furfural (by evaporation from the top of the distillation column) to leave behind the a liquid aromatic phase again affords the advantage that the formation of furfural condensation products is minimized, leading to a higher purity product.

In a preferred embodiment the process according to the invention further comprises a step wherein the retrieved furfural is converted into a furfural derivative, such as for example methyltetrahydrofuran or methylfuran, and wherein this furfural derivative is blended with one or more other fuel components to produce a biofuel.

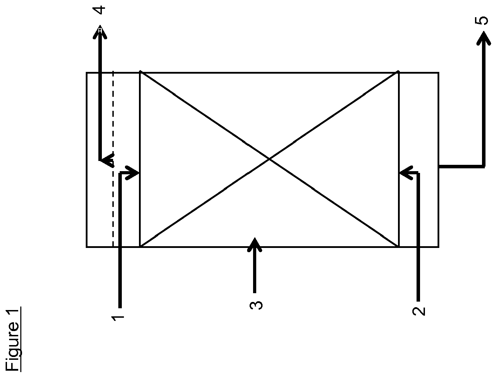

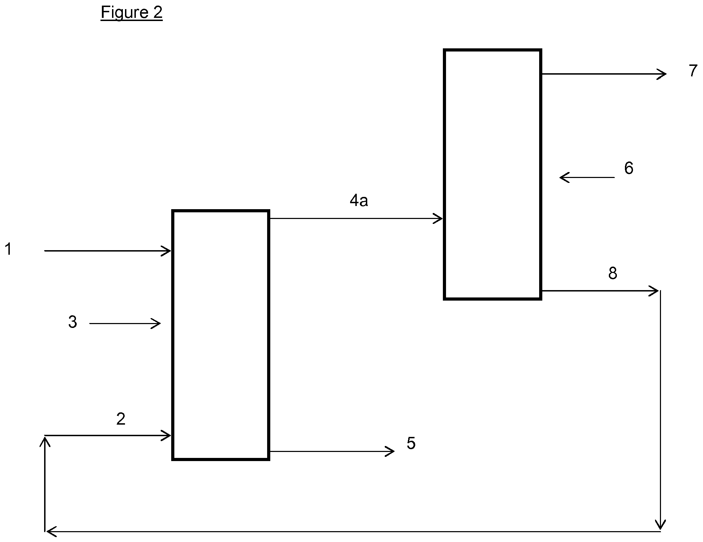

Figures 1 and 2 show process schemes for two embodiments of the process according to the invention.

In the embodiment shown in Figure 1, a liquid aqueous phase stream, comprising furfural and at least one of levulinic acid, formic acid and acetic acid, which stream is obtained from an acid-catalyzed hydrolysis of lignocellulosic material, (flow 1), is directed to extraction column (3). An liquid aromatic phase (2) comprising aromatic hydrocarbon compounds including a mixture of a C₁₋₆ alkyl substituted naphthalene with a C₁₋₆ alkyl substituted benzene or comprising a mixture of naphthalene and 1-methylnaphthalene is also introduced into the extraction column (3). In the embodiment shown, the liquid aqueous phase and the liquid aromatic phase are introduced into the extraction column at opposite ends and flow counter-currently with respect to each other, the higher density liquid stream (liquid aqueous phase) entering at the top of the column and the lower density liquid stream (liquid aromatic phase) entering at the bottom of the extraction column (3). In extraction column (3), furfural is extracted from the liquid aqueous phase into the liquid aromatic phase to provide a liquid aqueous phase depleted in furfural and an liquid organic phase comprising furfural and one or more aromatic hydrocarbon compounds. These two phases have different densities and may be separated by gravitational separation as shown in Figure 1 where the heavier liquid aqueous phase depleted in furfural is withdrawn from the bottom of the extraction column (flow 5) and the lighter liquid organic phase enriched in furfural (flow 4) is removed as the overhead product.

In the embodiment shown in Figure 2, the extraction process is performed using an aromatic hydrocarbon compound having a higher boiling point than furfural.

The liquid organic phase enriched in furfural and comprising this aromatic hydrocarbon compound having a higher boiling point than furfural (4a) is then supplied to a distillation column (6) where the lower boiling point (and therefore more volatile) furfural is separated from the higher boiling point aromatic hydrocarbon compound by distillation. Furfural is obtained as a vapour (flow 7) in the overhead of the distillation column and the vapour is condensed to form liquid furfural (not shown). The aromatic hydrocarbon compound is removed from the bottom of the distillation column (flow 8) and recycled back to the extraction column as (part of) the liquid aromatic phase (2).

Throughout the description and claims of this specification, the words "comprise" and "contain" and variations of the words, for example "comprising" and "comprises", mean "including but not limited to", and do not exclude other moieties, additives, components, integers or steps.

### EXAMPLES

The invention will now be further illustrated by means of the following non-limiting examples.

Abbreviations used in the examples below include:
- AR =: Liquid Aromatic Phase
- AQ =: Liquid Aqueous Phase
- OR =: Liquid Organic Phase
- FF =: Furfural
- LA =: Levulinic Acid
- AA =: Acetic Acid
- FA =: Formic Acid
- HS =: Hydrolysate feed solution
- T =: Temperature

### Example 1 Extraction of furfural using alkylbenzene as liquid aromatic phase:

### (i)Hydrolysate feed solution preparation

A 1000 ml artificial lignocellulosic hydrolysate feed solution (also referred to as "hydrolysate" or HS) was prepared according to Table 1.

**Table 1: Hydrolysate feed solution for example 1**

| Component | Weight in (g) | Grade (%) | wt% |
|---|---|---|---|
| Water | 816.21 | 100 | 81.2 |
| Furfural (FF) | 26.30 | 100 | 2.6 |
| Levulinic acid (LA) | 96.01 | 98 | 9.4 |
| Formic acid (FA) | 36.00 | 98 | 3.5 |
| Acetic acid (AA) | 33.64 | 100 | 3.3 |
| **Total** | 1008.16 | | 100.0 |

### (ii) Extraction using butylbenzene, pentylbenzene or hexylbenzene as the liquid aromatic phase:

The hydrolysate (as liquid aqueous phase (AQ)) and an aromatic hydrocarbon compound (forming the liquid aromatic phase (AR)) as indicated in tables 2a, 2b and 2c were mixed together in the following weight portions: 50:25, 50:50 and 50:100 g. The extraction took place in a 500 ml bottle on a stirring device at room temperature (about 20°C) for 30 minutes. An emulsion was obtained.

The emulsion was poured in a separation funnel where a liquid aqueous phase and a liquid organic phase separated quickly, with the formed liquid aqueous phase being the more dense. The two phases were collected in labeled bottles and their contents analyzed.

The above experiments were repeated at 60°C, using a boiling flask with a reflux cooler attached. The temperature was maintained by putting the flask in an oil bath with a temperature controller connected to a hotplate heating the oil.

### Analysis

A mass balance was made for every extraction. Also the distribution ratio (Kd) of furfural (FF) was calculated by dividing the furfural content in the liquid organic phase (wt/wt%) by the furfural content in the liquid aqueous phase (wt/wt%). The distribution ratios (Kd) of levulinic acid (LA), acetic acid (AA) and formic acid (FA) were calculated in an analogous manner.

Water content was determined using a Titroline KF from Schott, a volumetric titrator using the Karl Fischer method. Levulinic acid, formic acid and acetic acid content of the liquid aqueous phase prepared at room temperature was analyzed using an HPLC (Agilent 1100 series). Organic Levulinic acid, formic acid and acetic acid content of the liquid organic phase from the experiments conducted at 60°C was analyzed using an IEC. Furfural and aromatic hydrocarbon compound content were measured using a GC (Trace GC Ultra).

### Results

The results obtained for extractions using butylbenzene, pentylbenzene and hexylbenzene as the liquid aromatic phase (AR) are presented in Tables 2a - 2c respectively.

The first row of each table shows the composition of the hydrolysate feed solution (HS) used. Subsequent rows show the results obtained for the different liquid phases after extraction in an alternative fashion (firstly the liquid aqueous phase (AQ) and secondly the liquid organic phase (OR)) for a given liquid aromatic phase/hydrolysate (AR/HS) weight ratio and temperature (T).

**Table 2a: butylbenzene as the liquid aromatic phase (AR)**

| | | | GC analysis | | | HPLC analysis | | | | | | KF |
|---|---|---|---|---|---|---|---|---|---|---|---|---|
| Phase | AR/HS ratio | T (°C) | AR (wt%) | FF (wt%) | Kd (FF) | LA (wt%) | Kd (LA) | FA (wt%) | Kd (FA) | AA (wt%) | Kd (AA) | Water (wt%) |
| HS | NA | NA | NA | 2.61 | NA | 9.33 | NA | 3.50 | NA | 3.34 | NA | 80.96 |
| AQ | 0.5 | 25 | 0.42 | 1.45 | | 9.51 | | 3.70 | | 3.57 | | 87.94 |
| OR | 0.5 | 25 | 94.26 | 2.18 | 1.50 | 0.09 | 0.01 | 0.00 | 0.00 | 0.00 | 0.00 | 0.04 |
| AQ | 1.0 | 25 | 0.01 | 1.08 | | 9.69 | | 3.78 | | 3.43 | | 87.69 |
| OR | 1.0 | 25 | 94.07 | 1.52 | 1.41 | 0.00 | 0.00 | 0.00 | 0.00 | 0.02 | 0.01 | 0.02 |
| AQ | 2.0 | 25 | 0.23 | 0.72 | | 9.08 | | 3.55 | | 3.27 | | 88.38 |
| OR | 2.0 | 25 | 95.24 | 0.98 | 1.36 | 0.29 | 0.03 | 0.04 | 0.01 | 0.09 | 0.03 | 0.03 |
| AQ | 0.5 | 60 | 0.09 | 1.52 | | 11.29 | | 3.90 | | 3.36 | | 83.90 |
| OR | 0.5 | 60 | 93.29 | 2.19 | 1.44 | 0.00 | 0.00 | 0.00 | 0.00 | 0.14 | 0.04 | 0.04 |
| AQ | 1.0 | 60 | 0.20 | 1.13 | | 11.00 | | 3.83 | | 3.29 | | 89.12 |
| OR | 1.0 | 60 | 94.34 | 1.54 | 1.36 | 0.00 | 0.00 | 0.00 | 0.00 | 0.17 | 0.05 | 0.02 |
| AQ | 2.0 | 60 | 0.31 | 0.74 | | 10.97 | | 3.76 | | 3.21 | | 86.68 |
| OR | 2.0 | 60 | 95.09 | 0.99 | 1.34 | 0.00 | 0.00 | 0.00 | 0.00 | 0.11 | 0.03 | 0.03 |

**Table 2b: pentylbenzene as the liquid aromatic phase (AR)**

| | | | GC analysis | | | HPLC analysis | | | | | | KF |
|---|---|---|---|---|---|---|---|---|---|---|---|---|
| Phase | AR/HS ratio | T (°C) | AR (wt%) | FF (wt%) | Kd (FF) | LA (wt%) | Kd (LA) | FA (wt%) | Kd (FA) | AA (wt%) | Kd (AA) | Water (wt%) |
| HS | NA | NA | NA | 2.61 | NA | 9.33 | NA | 3.50 | NA | 3.34 | NA | 80.96 |
| AQ | 0.5 | 25 | 0.29 | 1.69 | | 9.62 | | 3.72 | | 3.55 | | 91.53 |
| OR | 0.5 | 25 | 92.07 | 2.05 | 1.21 | 0.00 | 0.00 | 0.00 | 0.00 | 0.00 | 0.00 | 0.06 |
| AQ | 1.0 | 25 | 0.06 | 1.23 | | 9.70 | | 3.78 | | 3.68 | | 92.33 |
| OR | 1.0 | 25 | 93.17 | 1.55 | 1.26 | 0.00 | 0.00 | 0.00 | 0.00 | 0.00 | 0.00 | 0.02 |
| AQ | 2.0 | 25 | 0.53 | 0.84 | | 9.60 | | 3.77 | | 3.51 | | 92.93 |
| OR | 2.0 | 25 | 92.72 | 0.99 | 1.18 | 0.04 | 0.00 | 0.00 | 0.00 | 0.00 | 0.00 | 0.01 |
| AQ | 0.5 | 60 | 0.10 | 1.59 | | 10.92 | | 3.79 | | 3.28 | | 83.23 |
| OR | 0.5 | 60 | 92.23 | 2.09 | 1.31 | 0.00 | 0.00 | 0.00 | 0.00 | 0.29 | 0.09 | 0.03 |
| AQ | 1.0 | 60 | 0.16 | 1.18 | | 10.98 | | 3.81 | | 3.29 | | 84.92 |
| OR | 1.0 | 60 | 91.49 | 1.49 | 1.26 | 0.00 | 0.00 | 0.00 | 0.00 | 0.14 | 0.04 | 0.03 |
| AQ | 2.0 | 60 | 0.24 | 0.80 | | 10.85 | | 3.79 | | 3.22 | | 85.93 |
| OR | 2.0 | 60 | 92.97 | 0.98 | 1.23 | 0.00 | 0.00 | 0.00 | 0.00 | 0.12 | 0.04 | 0.02 |

**Table 2c: hexylbenzene as the liquid aromatic phase (AR)**

| | | | GC analysis | | | HPLC analysis | | | | | | KF |
|---|---|---|---|---|---|---|---|---|---|---|---|---|
| Phase | AR/HS ratio | T (°C) | AR (wt%) | FF (wt%) | Kd (FF) | LA (wt%) | Kd (LA) | FA (wt%) | Kd (FA) | AA (wt%) | Kd (AA) | Water (wt%) |
| HS | NA | NA | NA | 2.61 | NA | 9.33 | NA | 3.50 | NA | 3.34 | NA | 80.96 |
| AQ | 0.5 | 25 | 0.41 | 1.74 | | 9.58 | | 3.75 | | 3.59 | | 91.86 |
| OR | 0.5 | 25 | 77.94 | 1.76 | 1.01 | 0.00 | 0.00 | 0.00 | 0.00 | 0.00 | 0.00 | 0.02 |
| AQ | 1.0 | 25 | 0.18 | 1.31 | | 9.64 | | 3.81 | | 3.58 | | 92.11 |
| OR | 1.0 | 25 | 89.71 | 1.33 | 1.02 | 0.00 | 0.00 | 0.00 | 0.00 | 0.00 | 0.00 | 0.02 |
| AQ | 2.0 | 25 | 0.35 | 0.90 | | 9.55 | | 3.73 | | 3.51 | | 89.19 |
| OR | 2.0 | 25 | 92.68 | 0.89 | 0.99 | 0.10 | 0.01 | 0.00 | 0.00 | 0.00 | 0.00 | 0.04 |
| AQ | 0.5 | 60 | 0.46 | 1.73 | | 11.36 | | 3.95 | | 3.43 | | 84.94 |
| OR | 0.5 | 60 | 90.52 | 1.81 | 1.05 | 0.00 | 0.00 | 0.00 | 0.00 | 0.00 | 0.00 | 0.02 |
| AQ | 1.0 | 60 | 0.30 | 1.32 | | 11.17 | | 3.88 | | 3.32 | | 84.62 |
| OR | 1.0 | 60 | 91.67 | 1.38 | 1.05 | 0.00 | 0.00 | 0.00 | 0.00 | 0.12 | 0.04 | 0.04 |
| AQ | 2.0 | 60 | 0.27 | 0.88 | | 10.96 | | 3.75 | | 3.20 | | 85.01 |
| OR | 2.0 | 60 | 91.18 | 0.90 | 1.02 | 0.00 | 0.00 | 0.00 | 0.00 | 0.13 | 0.04 | 0.03 |

From the results presented it can be seen that all three alkylbenzenes as liquid aromatic phase were found to have high selectivity for furfural, with Kds of between 1.46 (butylbenzene, 25°C) and 1.01 (hexylbenzene, 25°C). The results obtained for extractions performed at 25°C and 60°C were similar, indicating that all three alkylbenzenes have low temperature sensitivity. The Kd values for the other components present in the hydrolysate (acetic acid, formic acid, levulinic acid) are all approximately equal to zero. The aromatic hydrocarbon compounds in the liquid aromatic phases used are therefore selective for furfural compared to the other components in the hydrolysate.

### (iii) Extraction using toluene as the liquid aromatic phase:

Extractions using toluene as the liquid aromatic phase at 25°C were performed as described above at several weight ratios of liquid aromatic phase (AR) to hydrolysate (HS) and the following results were obtained:

**Table 3a: toluene as the liquid aromatic phase (AR) at 25°C**

| | AR/HS wt ratio = | | 0.5 |
|---|---|---|---|
| Component | AQ (wt%) | OR (wt%) | Kd |
| Furfural, GLC/FID | 0.895 | 2.700 | 3.017 |
| Levulinic acid, HPLC | 9.565 | 0.074 | 0.008 |
| Formic Acid, HPLC | 4.395 | 0.000 | 0.000 |
| Acetic acid, HPLC | 2.760 | 0.000 | 0.000 |

**Table 3b: toluene as the liquid aromatic phase (AR) at 25°C**

| | AR/HS wt ratio = | | 1.0 |
|---|---|---|---|
| Component | AQ (wt%) | OR (wt%) | Kd |
| Furfural, GLC/FID | 0.495 | 1.800 | 3.636 |
| Levulinic acid, HPLC | 9.525 | 0.144 | 0.015 |
| Formic Acid, HPLC | 4.400 | 0.000 | 0.000 |
| Acetic acid, HPLC | 2.685 | 0.088 | 0.033 |

**Table 3c: toluene as the liquid aromatic phase (AR) at 25°C**

| | AR/HS wt ratio = | | 2.0 |
|---|---|---|---|
| Component | AQ (wt%) | OR (wt%) | Kd |
| Furfural, GLC/FID | 0.310 | 1.075 | 3.468 |
| Levulinic acid, HPLC | 9.410 | 0.243 | 0.026 |
| Formic Acid, HPLC | 4.410 | 0.000 | 0.000 |
| Acetic acid, HPLC | 2.575 | 0.128 | 0.050 |

From the results, it can be seen that toluene at 25°C is also selective for furfural. A distribution coefficient (Kd) for furfural of greater than 3 is obtained but the Kd values for the other hydrolysate components are all approximately equal to zero.

Although toluene as liquid aromatic phase selectively extracts furfural from the hydrolysate (as liquid aqueous phase), more energy will be required as toluene has a lower boiling point than furfural which means that in order to recover the furfural, the toluene volume will have to be evaporated. When using toluene as liquid aromatic phase, the furfural will be taken from the bottom of the distillation column rather than the top (as with the longer chain alkyl benzenes) and so the furfural will be present in a higher concentration and at higher temperatures than if a higher boiling point aromatic hydrocarbon compound was used, which conditions tend to favor the production of undesired furfural condensation products.

### Example 2 Extraction of furfural using naphthalene in methylnaphthalene

### (i) Hydrolysate feed solution preparation

A 200 ml artificial lignocellulosic hydrolysate feed solution (also referred to as "hydrolysate" or HS) was prepared having the following composition (see table 4) :

**Table 4 Hydrolysate feed solution for example 2**

| Component | Weigh in (g) | Grade (%) | wt% |
|---|---|---|---|
| Water | 163.15 | 100 | 81.9 |
| Furfural (FF) | 5.41 | 100 (Merck) | 2.7 |
| Levulinic acid (LA) | 19.20 | 98 (Sigma Aldrich) | 9.4 |
| Formic acid (FA) | 6.78 | 98 (Merck) | 3.3 |
| Acetic acid (AA) | 5.47 | 100 (Merck) | 2.7 |
| **Total** | 200.01 | | 100.0 |

### (ii) Preparation of the liquid aromatic phase

A solution of about 20wt% naphthalene in methylnaphthalene was prepared having the following composition (see table 5):

**Table 5: Liquid aromatic phase**

| Component | Weigh in (g) | Grade (wt%) | wt% |
|---|---|---|---|
| Methylnaphthalene | 149.29 | 94 (Merck) | 77.7 |
| Naphthalene | 40.61 | 99 (Sigma Aldrich) | 22.3 |
| **Total** | 189.90 | | 100.0 |

### (iii) Extraction

The hydrolysate (as liquid aqueous phase) and liquid aromatic phase were mixed together in the following weight proportions: 50:25, 50:50 and 50:100. The extraction was performed in a boiling flask with a reflux cooler, placed in an oil bath on a stirring device, for a period of 30 minutes at a constant temperature of 60°C. An emulsion was obtained.

The emulsion was poured in a separation funnel where a liquid aqueous phase and a liquid organic phase separated quickly, with the formed liquid aqueous phase being the more dense. The two phases were collected in labeled bottles and their contents analyzed.

### (iv) Analysis

The distribution ratio (Kd) of furfural, water content, organic acid content, furfural content and aromatic hydrocarbon compound content were determined according to the methods used in Example 1.

### (v) Results

The results obtained using 21.4% naphthalene in methylnaphthalene as a liquid aromatic phase are presented in Table 6 below.

As for Tables 2a-2c above, the first row of each table shows the composition of the hydrolysate feed solution (HS) used. Subsequent rows show the results obtained for the different liquid phases after extraction in an alternative fashion (firstly the liquid aqueous phase (AQ) and secondly the liquid organic phase (OR)) for a given liquid aromatic phase/hydrolysate (AR/HS) weight ratio and temperature (T).

The Kd obtained for naphthalene in methylnaphthalene (Kd 3.0) is similar to that obtained when toluene is used and is approximately double the value obtained for butylbenzene as liquid aromatic phase. The Kd values of the other components present in the hydrolysate feed solution are zero or almost zero, demonstrating that naphthalene in methylnaphthalene is selective for furfural extraction.

**Table 6: solution of naphthalene in methylnaphthalene as the liquid aromatic phase (AR)**

| | | | GC analysis | | | HPLC analysis | | | | | | KF |
|---|---|---|---|---|---|---|---|---|---|---|---|---|
| Phase | AR/HS ratio | T (°C) | AR (wt%) | FF (wt%) | Kd (FF) | LA (wt%) | Kd (LA) | FA (wt%) | Kd (FA) | AA (wt%) | Kd (AA) | Water (wt%) |
| HS | NA | NA | NA | 2.70 | NA | 9.41 | NA | 3.32 | NA | 2.73 | NA | 81.57 |
| AQ | 0.5 | 60 | 0.87 | 1.07 | NA | 9.44 | NA | 3.56 | NA | 2.94 | NA | 88.71 |
| OR | 0.5 | 60 | 96.88 | 3.27 | 3.05 | 0.59 | 0.06 | 0.00 | 0.00 | 0.00 | 0.00 | 1.19 |
| AQ | 1.0 | 60 | 1.76 | 0.70 | NA | 9.29 | NA | 3.49 | NA | 2.87 | NA | 92.10 |
| OR | 1.0 | 60 | 95.85 | 2.01 | 2.89 | 0.56 | 0.06 | 0.00 | 0.00 | 0.01 | 0.00 | 1.44 |
| AQ | 2.0 | 60 | 1.10 | 0.40 | NA | 9.35 | NA | 3.57 | NA | 2.85 | NA | 92.84 |
| OR | 2.0 | 60 | 98.61 | 1.17 | 2.93 | 0.38 | 0.04 | 0.01 | 0.00 | 0.05 | 0.02 | 0.81 |

### Comparative example A: Extraction of furfural using 2-methyl tetrahydrofuran (2-MTHF)or cyclohexanone

### (i) Hydrolysate feed solution preparation

Artificial lignocellulosic hydrolysate feed solution (also referred to as "hydrolysate" or HS) was prepared having the compositions as indicated in Tables 7a, 7b, 7c and 7d.

### (ii) Extraction using methyl tetrahydrofuran or cyclohexanone:

The hydrolysate (as liquid aqueous phase (AQ)) and 2-methyl tetrahydrofuran (2-MTHF) or cyclohexanone were mixed together in a weight ratio of 2-MTHF or cyclohexanone to hydrolysate as indicated in tables 7a, 7b, 7c and 7d. The extraction took place in a 500 ml bottle on a stirring device at room temperature (about 20°C) for 30 minutes. An emulsion was obtained.

The emulsion was poured in a separation funnel where a liquid aqueous phase (AQ) and a liquid organic phase (OR) separated quickly, with the formed liquid aqueous phase being the more dense. The two phases were collected in labeled bottles and their contents analyzed.

For cyclohexanone the experiment was repeated at 60°C, using a boiling flask with a reflux cooler attached. The temperature was maintained by putting the flask in an oil bath with a temperature controller connected to a hotplate heating the oil.

### (iv) Analysis

The distribution ratio (Kd) of furfural, levulinic acid, formic acid and sulphuric acid and water content, levulinic acid content, formic acid content, sulphuric acid content, furfural content were determined according to the methods used in Example 1.

### (v) Results

The results obtained using 2-MTHF and cyclohexanone to extract furfural are presented in Tables 7a, 7b, 7c and 7d below. As can be seen from Tables 7a, 7b, 7c and 7d, extraction with 2-MTHF or cyclohexanone does not selectively extract the furfural but also extracts considerable amounts of levulinic acid and formic acid.

**Table 7a: 2-Methyl tetrahydrofuran (2-MTHF) to extract furfural at a temperature of 20°C and a weight ratio of 2-MTHF to HS of 1.**

| | HS | AQ | OR | Kd |
|---|---|---|---|---|
| | % wt. | % wt. | % wt. | |
| 2-MTHF | | 11.52 | 78.42 | |
| Water | 78.96 | 80.69 | 9. 67 | |
| Levulinic acid | 10.00 | 3.27 | 6.00 | 1.83 |
| Furfural | 5.04 | 0.58 | 4.06 | 7.03 |
| Formic acid | 3.00 | 0.87 | 1.80 | 2.08 |
| Sulphuric acid | 3.01 | 3.08 | 0.05 | 0.01 |
| total | 100.00 | 100.00 | 100.00 | |

**Table 7b: 2-Methyl tetrahydrofuran (2-MTHF) to extract furfural at a temperature of 20°C and a weight ratio of 2-MTHF to HS of 0.50.**

| | HS | AQ | OR | Kd |
|---|---|---|---|---|
| | % wt. | % wt. | % wt. | |
| 2-MTHF | | 12.13 | 66.92 | |
| Water | 78.86 | 76.22 | 13.12 | |
| Levulinic acid | 9.98 | 5.47 | 10.06 | 1.84 |
| Furfural | 5.09 | 1.19 | 7.20 | 6.07 |
| Formic acid | 3.04 | 1.47 | 2.57 | 1.76 |
| Sulphuric acid | 3.03 | 3.52 | 0.13 | 0.04 |
| total | 100.00 | 100.00 | 100.00 | |

**Table 7c: Cyclohexanone to extract furfural at a temperature of 20°C and a weight ratio of cyclohexanone to HS of 1.34.**

| | HS | AQ | OR | Kd |
|---|---|---|---|---|
| | % wt. | % wt. | % wt. | |
| Cyclohexanone | | 7.45 | 80.50 | |
| Water | 81.57 | 83.78 | 9.68 | |
| Levulinic acid | 9.38 | 3.33 | 5.78 | 1.73 |
| Furfural | 2.60 | 0.19 | 2.08 | 10.88 |
| Formic acid | 3.27 | 1.47 | 1. 96 | 1.34 |
| Sulphuric acid | 3.19 | n.d. | n.d. | |
| total | 100.00 | 96.22 | 100.00 | |

**Table 7d: Cyclohexanone to extract furfural at a temperature of 60°C and a weight ratio of cyclohexanone to HS of 1.40.**

| | HS | AQ | OR | Kd |
|---|---|---|---|---|
| | % wt. | % wt. | % wt. | |
| Cyclohexanone | | 7.45 | 80.24 | |
| Water | 81.97 | 83.78 | 10.05 | |
| Levulinic acid | 9.43 | 3.33 | 5.81 | 1.73 |
| Furfural | 2.59 | 0.19 | 2.01 | 10.88 |
| Formic acid | 3.29 | 1.47 | 1.88 | 1.34 |
| Sulphuric acid | 2.72 | n.d. | n.d. | |
| total | 100.00 | 96.22 | 100.00 | |

As illustrated above, surprisingly aromatic hydrocarbon compounds can be used to extract furfural selectively from a liquid aqueous phase comprising furfural and one or more organic acids (for example formed by acid catalyzed hydrolysis of biomass), whereas other hydrocarbon compounds are not suitable for such use.

## Claims

1. A process for separating furfural from a liquid aqueous phase comprising furfural and one or more organic acids chosen from the group of levulinic acid, acetic acid, formic acid and mixtures thereof, which process comprises a step
a) extracting furfural from the liquid aqueous phase into a liquid aromatic phase to obtain a liquid organic phase comprising furfural, **characterized in that** the liquid aromatic phase comprises aromatic hydrocarbon compounds including a mixture of a C₁₋₆ alkyl substituted naphthalene with a C₁₋₆ alkyl substituted benzene or comprising a mixture of naphthalene and 1-methylnaphthalene.

2. The process according to claim 1, wherein the process further comprises a step
b) retrieving furfural from the liquid organic phase obtained in step a) to obtain furfural.

3. The process according to claim 2, wherein the process further comprises a step
c) converting the furfural obtained in step b) in a furfural derivative and blending the furfural derivative with one or more other fuel components to produce a biofuel.

4. The process according to any of claims 1 to 3, wherein the liquid aqueous phase is produced by acid catalyzed hydrolysis of lignocellulosic material.

5. The process according to any of claims 1 to 4, wherein the liquid aqueous phase comprises more than or equal to 0.1 wt% and less than or equal to 30 wt% of furfural based on the total weight of the liquid aqueous phase.

6. The process according to any of claims 1 to 5, wherein the liquid aqueous phase comprises from more than or equal to 0.1 wt% and less than or equal to 30 wt% of one or more organic acids based on the total weight of the liquid aqueous phase.

7. The process according to any of claims 1 to 6, wherein the liquid aromatic phase comprises one or more aromatic hydrocarbon compounds having a higher boiling point than furfural.

8. The process according to any of claims 1 to 7, wherein the ratio of the liquid aqueous phase to the liquid aromatic phase is in the range of from 2:1 to 1:5.

9. The process according to any of claims 1 to 8, wherein the process further comprises retrieving one or more aromatic hydrocarbon compounds from the liquid organic phase and recycling the retrieved aromatic hydrocarbon compound(s) to step a).

10. Use of aromatic hydrocarbon compounds including a mixture of a C₁₋₆ alkyl substituted naphthalene with a C₁₋₆ alkyl substituted benzene or comprising a mixture of naphthalene and 1-methylnaphthalene to extract furfural selectively from a liquid aqueous phase comprising furfural and one or more organic acids chosen from the group of levulinic acid, acetic acid, formic acid and mixtures thereof, formed by acid catalysed hydrolysis of biomass.

## Patentansprüche

1. Verfahren zur Abtrennung von Furfural aus einer flüssigen wässrigen Phase, die Furfural und eine oder mehrere organische Säuren aus der Gruppe Lävulinsäure, Essigsäure, Ameisensäure und Mischungen davon umfasst, wobei das Verfahren den folgenden Schritt umfasst:
a) Extrahieren von Furfural aus der flüssigen wässrigen Phase in eine flüssige aromatische Phase zum Erhalt einer flüssigen organischen Phase, die Furfural umfasst, **dadurch gekennzeichnet, dass** die flüssige aromatische Phase aromatische Kohlenwasserstoffverbindungen, die eine Mischung aus einem C₁₋₆-alkylsubstituierten Naphthalin mit einem C₁₋₆-alkylsubstituierten Benzol enthalten oder eine Mischung von Naphthalin und 1-Methylnaphthalin umfassen, umfasst.

2. Verfahren nach Anspruch 1, bei dem das Verfahren ferner den folgenden Schritt umfasst:
b) Rückgewinnen von Furfural aus der in Schritt a) erhaltenen flüssigen organischen Phase zum Erhalt von Furfural.

3. Verfahren nach Anspruch 2, bei dem das Verfahren ferner den folgenden Schritt umfasst:
c) Umwandeln des in Schritt b) erhaltenen Furfurals in ein Furfuralderivat und Mischen des Furfuralderivats mit einer oder mehreren Brennstoffkomponenten zur Herstellung eines Biobrennstoffs.

4. Verfahren nach einem der Ansprüche 1 bis 3, bei dem die flüssige wässrige Phase durch säurekatalysierte Hydrolyse von lignocellulosehaltigem Material erzeugt wird.

5. Verfahren nach einem der Ansprüche 1 bis 4, bei dem die flüssige wässrige Phase größer gleich 0,1 Gew.-% und kleiner gleich 30 Gew.-% Furfural, bezogen auf das Gesamtgewicht der flüssigen wässrigen Phase, umfasst.

6. Verfahren nach einem der Ansprüche 1 bis 5, bei dem die flüssige wässrige Phase größer gleich 0,1 Gew.-% und kleiner gleich 30 Gew.-% einer oder mehrerer organischer Säuren, bezogen auf das Gesamtgewicht der flüssigen wässrigen Phase, umfasst.

7. Verfahren nach einem der Ansprüche 1 bis 6, bei dem die flüssige aromatische Phase eine oder mehrere aromatische Kohlenwasserstoffverbindungen mit einem höheren Siedepunkt als Furfural umfasst.

8. Verfahren nach einem der Ansprüche 1 bis 7, bei dem das Verhältnis von flüssiger wässriger Phase zu flüssiger aromatischer Phase im Bereich von 2:1 bis 1:5 liegt.

9. Verfahren nach einem der Ansprüche 1 bis 8, bei dem das Verfahren ferner das Rückgewinnen einer oder mehrerer aromatischer Kohlenwasserstoffverbindungen aus der flüssigen organischen Phase und das Rezyklieren der zurückgewonnenen aromatischen Kohlenwasserstoffverbindung bzw. der zurückgewonnenen aromatischen Kohlenwasserstoffverbindungen in Schritt a) umfasst.

10. Verwendung von aromatischen Kohlenwasserstoffverbindungen, die eine Mischung aus einem C₁₋₆-alkylsubstituierten Naphthalin mit einem C₁₋₆-alkylsubstituierten Benzol enthalten oder eine Mischung von Naphthalin und 1-Methylnaphthalin umfassen, zum selektiven Extrahieren von Furfural aus einer durch säurekatalysierte Hydrolyse von Biomasse gebildeten flüssigen wässrigen Phase, die Furfural und eine oder mehrere organische Säuren aus der Gruppe Lävulinsäure, Essigsäure, Ameisensäure und Mischungen davon umfasst.

## Revendications

1. Procédé de séparation de furfural d'une phase aqueuse liquide comprenant du furfural et un ou plusieurs acides organiques choisis dans le groupe de l'acide lévulinique, l'acide acétique, l'acide formique et les mélanges de ceux-ci, lequel procédé comprend une étape
a) extraire du furfural de la phase aqueuse liquide dans une phase aromatique liquide pour obtenir une phase organique liquide comprenant du furfural, **caractérisé en ce que** la phase aromatique liquide comprend des composés hydrocarbonés aromatiques comportant un mélange d'un naphtalène à substitution alkyle en C₁₋₆ avec un benzène à substitution alkyle en C₁₋₆ ou comprenant un mélange de naphtalène et de 1-méthylnaphtalène.

2. Procédé selon la revendication 1, le procédé comprenant en outre une étape
b) récupérer du furfural à partir de la phase organique liquide obtenue à l'étape a) pour obtenir du furfural.

3. Procédé selon la revendication 2, le procédé comprenant en outre une étape
c) transformer le furfural obtenu à l'étape b) en un dérivé de furfural et mélanger le dérivé de furfural avec un ou plusieurs autres constituants de carburant pour produire un biocarburant.

4. Procédé selon l'une quelconque des revendications 1 à 3, dans lequel la phase aqueuse liquide est produite par hydrolyse à catalyse acide de matière lignocellulosique.

5. Procédé selon l'une quelconque des revendications 1 à 4, dans lequel la phase aqueuse liquide comprend au moins 0,1% en poids et au maximum 30 % en poids de furfural, rapporté au poids total de la phase aqueuse liquide.

6. Procédé selon l'une quelconque des revendications 1 à 5, dans lequel la phase aqueuse liquide comprend au moins 0,1% en poids et au maximum 30 % en poids d'un ou plusieurs acides organiques, rapporté au poids total de la phase aqueuse liquide.

7. Procédé selon l'une quelconque des revendications 1 à 6, dans lequel la phase aromatique liquide comprend un ou plusieurs composés hydrocarbonés aromatiques ayant un plus haut point d'ébullition que le furfural.

8. Procédé selon l'une quelconque des revendications 1 à 7, dans lequel le rapport de la phase aqueuse liquide à la phase aromatique liquide se situe dans la gamme de 2:1 à 1:5.

9. Procédé selon l'une quelconque des revendications 1 à 8, le procédé comprenant en outre la récupération d'un ou plusieurs composés hydrocarbonés aromatiques à partir de la phase organique liquide et le recyclage du ou des composés hydrocarbonés aromatiques récupérés à l'étape a).

10. Utilisation de composés hydrocarbonés aromatiques comportant un mélange d'un naphtalène à substitution alkyle en C₁₋₆ avec un benzène à substitution alkyle en C₁₋₆ ou comprenant un mélange de naphtalène et de 1-méthylnaphtalène pour extraire sélectivement du furfural d'une phase aqueuse liquide comprenant du furfural et un ou plusieurs acides organiques choisis dans le groupe de l'acide lévulinique, l'acide acétique, l'acide formique et les mélanges de ceux-ci, formée par hydrolyse à catalyse acide de biomasse.
